# EUROPÄISCHE PATENTANMELDUNG

(11) **EP 4 059 914 A1**
(43) Veröffentlichungstag der Anmeldung: **21.09.2022**
(21) Anmeldenummer: 21163483.7
(22) Anmeldetag: 18.03.2021
(51) Int. Cl.: C07B 41/06, C07C 45/00, C07F 9/6574

(54) **BISPHOSPHITLIGANDEN AUF BASIS VON BENZPINAKOL UND 1,3-PROPANDIOL**

(71) Anmelder: Evonik Operations GmbH, 45128 Essen (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); MARKOVIC, Ana, 45721 Haltern am See (DE); KUCMIERCZYK, Peter, 44628 Herne (DE); KNOSSALLA, Johannes, 46514 Gahlen (DE); SELENT, Detlef, 18059 Rostock (DE); BÖRNER, Armin, 18059 Rostock (DE); ROMEIKE, Kerstin, 18109 Rostock (DE)
(74) Vertreter: Evonik Patent Association

(57) **Zusammenfassung**

Bisphosphitliganden auf Basis von Benzpinakol und 1,3-Propandiol, sowie deren Verwendung in der Hydroformylierung.

## Beschreibung

Die vorliegende Erfindung betrifft Bisphosphitliganden auf Basis von Benzpinakol und 1,3-Propandiol, sowie deren Verwendung in der Hydroformylierung.

In WO 2008/071508 A1 wird ein Verfahren zur Hydroformylierung unter Einsatz von Bisphosphitliganden beschrieben. Unter anderem wird der Einsatz des Liganden (D-1) beschrieben.

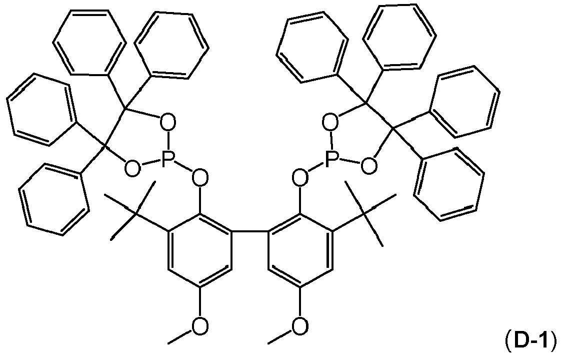

Die technische Aufgabe, welche der vorliegenden Erfindung zugrunde lag, ist die Bereitstellung neuer Verbindungen, welche gegenüber dem aus dem Stand der Technik bekannten Verbindungen bei der Hydroformylierung von Olefinen eine gesteigerte Ausbeute liefern.

Diese Aufgabe wird gelöst durch eine Verbindung nach Anspruch 1.

Verbindung gemäß Formel (I): 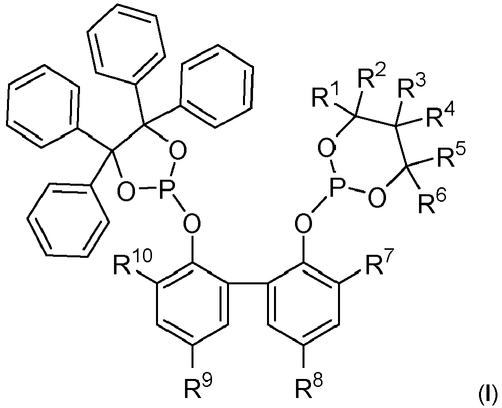 wobei
R¹, R², R³, R⁴, R⁵, R⁶, R⁷, R⁸, R⁹, R¹⁰ jeweils unabhängig voneinander ausgewählt sind aus: - H, -(C₁-C₁₂)-Alkyl, -O-(C₁-C₁₂)-Alkyl, -(C₆-C₁₂)-Aryl.

Der Begriff -(C₁-C₁₂)-Alkyl bzw. -O-(C₁-C₁₂)-Alkyl umfasst geradkettige und verzweigte Alkylgruppen mit 1 bis 12 Kohlenstoffatomen. Vorzugsweise handelt es sich dabei um -(C₁-C₈)-Alkylgruppen bzw. -O-(C₁-C₈)-Alkylgruppen, besonders bevorzugt um -(C₁-C₄)-Alkylgruppen bzw. -O-(C₁-C₄)-Alkylgruppen.

Der Begriff (C₆-C₁₂)-Aryl umfasst mono- oder polycyclische aromatische Kohlenwasserstoffreste mit 6 bis 12 Kohlenstoffatomen. Vorzugsweise handelt es sich dabei um -(C₆)-Aryl, also Phenyl.

In einer Ausführungsform stehen R⁷ und R¹⁰ für -(C₁-C₁₂)-Alkyl.

In einer Ausführungsform stehen R⁷ und R¹⁰ für -*^{ter}*Bu.

In einer Ausführungsform sind R⁸, R⁹ ausgewählt aus: -(C₁-C₁₂)-Alkyl, -O-(C₁-C₁₂)-Alkyl.

In einer Ausführungsform stehen R⁸ und R⁹ für -OCH₃ oder-*^{ter}*Bu.

In einer Ausführungsform stehen R⁸ und R⁹ für -OCH₃.

In einer Ausführungsform sind R¹, R², R³, R⁴, R⁵, R⁶ ausgewählt aus -H, -(C₁-C₁₂)-Alkyl, -(C₆-C₁₂)-Aryl.

In einer Ausführungsform stehen R¹, R², R⁵, R⁶ für-H.

In einer Ausführungsform sind R³, R⁴ ausgewählt aus -H, -CH₃, -Phenyl.

In einer Ausführungsform weist die Verbindung eine der Strukturen (1) oder (2) auf:

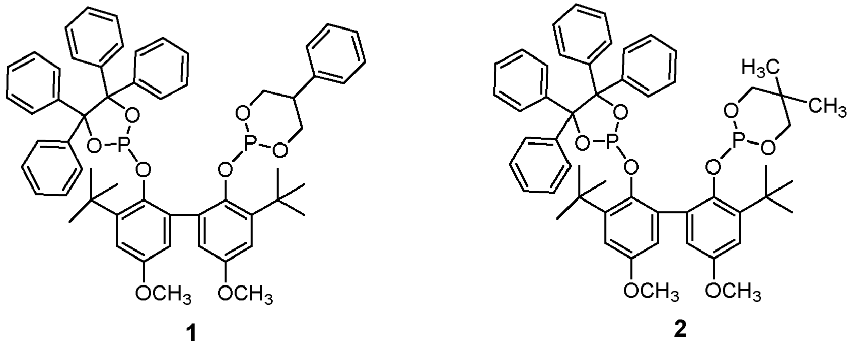

Neben der Verbindung an sich, wird auch ein Verfahren beansprucht, in welchen die Verbindung zum Einsatz kommt.

Verfahren umfassend die Verfahrensschritte:
a) Vorlegen einer ethylenisch ungesättigten Verbindung;
b) Zugabe einer wie zuvor beschriebenen Verbindung
   und einer Substanz, welche Rh umfasst;
c) Zuführen von H₂ und CO,
d) Erwärmen des Reaktionsgemisches aus a) bis c), wobei die ethylenisch ungesättigten Verbindung zu einem Aldehyd umgesetzt wird.

Hierbei können die Verfahrensschritte a), b) und c) in beliebiger Reihenfolge erfolgen. Üblicherweise erfolgt die Zugabe von CO jedoch, nachdem die Reaktionspartner in den Schritten a) und b) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

In einer Variante des Verfahrens umfasst die ethylenisch ungesättigte Verbindung keine weiteren funktionellen Gruppen außer Kohlenstoff-Kohlenstoff-Doppelbindungen.

In einer Variante des Verfahrens ist die ethylenisch ungesättigte Verbindung ausgewählt aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

In einer Variante des Verfahrens ist die Substanz, welche Rh umfasst, ausgewählt aus: Rh(acac)(CO)₂, [(acac)Rh(COD)] (Umicore, acac = Acetylacetonat-Anion; COD = 1,5-Cyclooctadien), Rh₄CO₁₂.

In einer Variante des Verfahrens erfolgt das Zuführen von CO in Verfahrensschritt c) mit einem Druck in dem Bereich von 1 bis 6 MPa (10 bis 60 bar).

In einer Variante des Verfahrens erfolgt das Erwärmen des Reaktionsgemisches in Verfahrensschritt d) auf eine Temperatur in dem Bereich von 80 °C bis 160 °C.

Im Folgenden soll die Erfindung anhand von Ausführungsbeispielen näher erläutert werden.

### Synthese 2-((3,3'-Di-tert-butyl-5,5'-dimethoxy-2'-((4,4,5,5-tetraphenyl-1,3,2-dioxaphospholan-2-yl)oxy)-[1,1'-biphenyl]-2-yl)oxy)-5-phenyl-1,3,2-dioxaphosphinan (1):

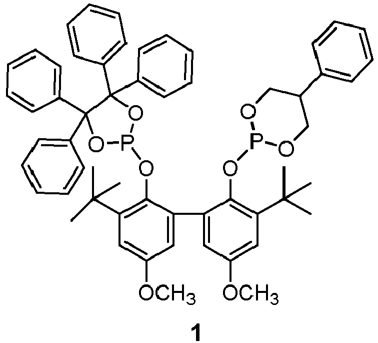

Zu einer Lösung von 4,4,5,5-Tetraphenyl-2-((3,3',5,5'-tetra-tert-butyl-2'-((dichlorophosphaneyl)oxy)-[1,1'-biphenyl]-2-yl)oxy)-1,3,2-dioxaphospholan (0,4361 g; 0,5108 mmol) in 5 ml Toluol wird bei Raumtemperatur tropfenweise eine Mischung von 2-Phenylpropan-1,3-diol (0,0777 g; 0,5108 mmol) und Triethylamin (0,28 ml) in 2 ml Toluol gegeben. Man rührt über Nacht, filtriert und engt das Filtrat im Vakuum zur Trockne ein. Der erhaltene Feststoff wird 2h bei 60°C/ 0,1mbar getrocknet anschließend 1 h mit 4 ml Heptan verrührt. Ausbeute: 0,37 g (0,396 mmol; 77%).

ESI-TOF HRMS: m/z=933,3684; [M⁺+H], ber. *m*/*z*=933,3679 und m/z=955,3489; [M⁺+Na], ber. m/z=955,3499.
³¹P-NMR (CD₂Cl₂): δ 116,3 (d, *J*_{PP}=17 Hz); 123,0 (d, *J*_{PP}=19 Hz); 145,3 (d, *J*_{PP}=17 Hz); 145,3 (d, *J*_{PP}=19Hz) ppm. 2 Isomere.
¹H-NMR (CD₂Cl₂): δ 1,07+1,12 (2s, 9H); 1,33+1,37 (2s, 9H); 3,54+3,57 (2s, 6H); 2,94-4,60 (m, 5H); 3,60+3,64 (2s, 6H); 6,58 (m, 2H); 6,78-7,33 (m, 27H) ppm.

### Synthese 2-((3,3'-Di-tert-butyl-5,5'-dimethoxy-2'-((4,4,5,5-tetraphenyl-1,3,2-dioxaphospholan-2-yl)oxy)-[1,1'-biphenyl]-2-yl)oxy)-5,5-dimethyl-1,3,2-dioxaphosphinane (2):

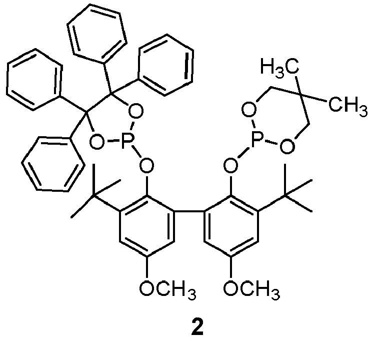

Zu einer Lösung von 4,4,5,5-Tetraphenyl-2-((3,3',5,5'-tetra-tert-butyl-2'-((dichlorophosphaneyl)oxy)-[1,1'-biphenyl]-2-yl)oxy)-1,3,2-dioxaphospholan (0,445 g; 0,5212 mmol) in 5 ml Toluol wird bei Raumtemperatur tropfenweise eine Mischung von von 2,2'-Dimethylpropan-1,3-diol (0,0543 g; 0,5212 mmol) und Triethylamin (0,29 ml) in 2 ml Toluol gegeben. Man rührt über Nacht, filtriert und engt das Filtrat im Vakuum zur Trockne ein. Der erhaltene Feststoff wird 2h bei 60°C/ 0,1mbar getrocknet. Ausbeute: 0,410 g (0,463 mmol, 89%).

Elementaranalyse (ber. für C₅₃H₅₈O₈P₂=884,98 g/Mol): C = 71,97 (71,93); H = 6,96 (6,61); P = 7,02 (7,00).

ESI-TOF HRMS: m/z=907,3514; [M⁺+Na], ber. *m*/*z*=907,3499.
³¹P-NMR (CD₂Cl₂): δ 116,6 (d, *J*_{PP}=19 Hz); 145,6 (d, *J*_{PP}=19 Hz) ppm.
¹H-NMR (CD₂Cl₂): δ 0,75 (s; 3H); 1,20 (s; 3H); 1,27 (s; 9H); 1,47 (s; 9H); 2,98 (m; 1H); 3,38 (s; 1H); 3,74 (s; 3H); 3,81 (s; 3H); 3,97 (m, 1H); 4,20 (m, 1H); 6,74 (m, 2H); 6,95-7,20 (m; 18H); 7,31 (m, 2H); 7,43 (m, 2H) ppm.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzten Olefin cis/trans-2-Penten (Aldrich) wurden über Natrium am Rückfluss erhitzt und unter Argon destilliert. Im Autoklaven wurden unter Argonatmosphäre Lösungen der Katalysatorvorstufe und des Liganden, jeweils in Toluol, gemischt. Als Katalysatorvorstufe kam [(acac)Rh(COD)] (Umicore, acac=Acetylacetonat-Anion; COD = 1,5-Cyclooctadien), zum Einsatz. Der Autoklav wurde bei 12 bar für den Enddruck von 20 bar unter Rühren (1500 U/min) aufgeheizt. Nach Erreichen der Reaktionstemperatur wurde das Olefin mit einem in der Druckpipette eingestellten Überdruck in den Autoklaven gepresst. Die Reaktion wurde bei konstantem Druck (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

Die Reaktion wurde mit den erfindungsgemäßen Verbindungen (1) und (2), sowie mit dem Vergleichsliganden (D-1) durchgeführt.

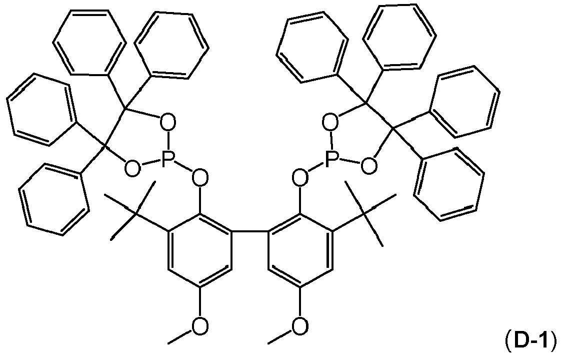

Reaktionsbedingungen:
Olefin: 2-Penten, Solvens: Toluol, Masseanteil Rhodium: 100 ppm, p: 20 bar, T: 120 °C, t: 4 h, Verhältnis Rh : Ligand = 1 : 2.

Die Ergebnisse sind in der nachfolgenden Tabelle zusammengestellt:

| Ligand | Ausbeute Aldehyd [%] |
|---|---|
| **1*** | 78 |
| **2*** | 74 |
| **D-1** | 14 |

| | |
|---|---|
| *erfindungsgemäße Verbindung | |

Wie die Versuchsergebnisse zeigen, wird die Aufgabe durch die erfindungsgemäßen Verbindungen gelöst.

## Patentansprüche

1. Verbindung gemäß Formel (I): 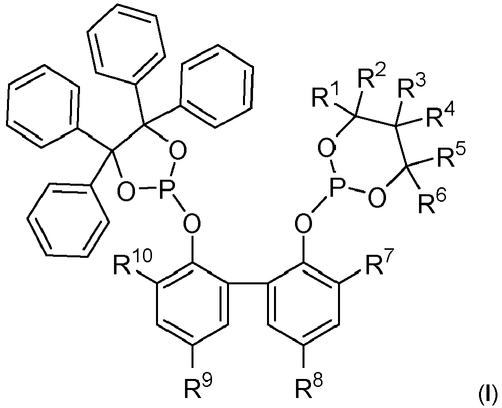 wobei
R¹, R², R³, R⁴, R⁵, R⁶, R⁷, R⁸, R⁹, R¹⁰ jeweils unabhängig voneinander ausgewählt sind aus: - H, -(C₁-C₁₂)-Alkyl, -O-(C₁-C₁₂)-Alkyl, -(C₆-C₁₂)-Aryl.

2. Verbindung nach Anspruch 1,
wobei R⁷ und R¹⁰ für -(C₁-C₁₂)-Alkyl stehen.

3. Verbindung nach einem der Ansprüche 1 oder 2,
wobei R⁷ und R¹⁰ für -*^{ter}*Bu stehen.

4. Verbindung nach einem der Ansprüche 1 bis 3,
wobei R⁸, R⁹ ausgewählt sind aus: -(C₁-C₁₂)-Alkyl, -O-(C₁-C₁₂)-Alkyl.

5. Verbindung nach einem der Ansprüche 1 bis 4,
wobei R⁸ und R⁹ für -OCH₃ oder-*^{ter}*Bu stehen.

6. Verbindung nach einem der Ansprüche 1 bis 5,
wobei R¹, R², R³, R⁴, R⁵, R⁶ ausgewählt sind aus -H, -(C₁-C₁₂)-Alkyl, -(C₆-C₁₂)-Aryl.

7. Verbindung nach einem der Ansprüche 1 bis 6,
wobei R¹, R², R⁵, R⁶ für -H stehen.

8. Verbindung nach einem der Ansprüche 1 bis 7,
wobei die Verbindung eine der Strukturen (1) oder (2) aufweist:

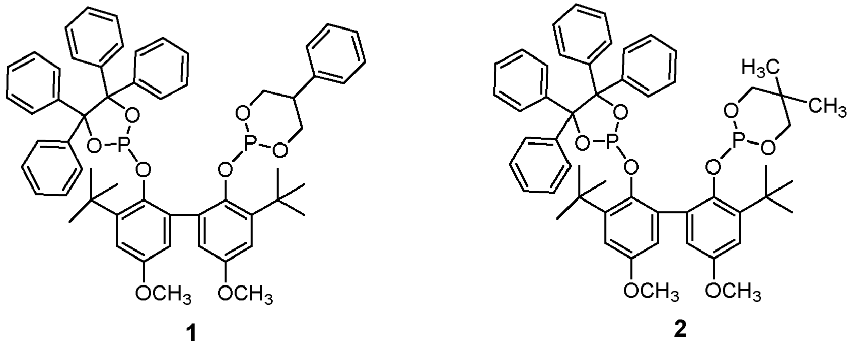

9. Verfahren umfassend die Verfahrensschritte:
a) Vorlegen einer ethylenisch ungesättigten Verbindung;
b) Zugabe einer Verbindung gemäß einem der Ansprüche 1 bis 8 und einer Substanz, welche Rh umfasst;
c) Zuführen von H₂ und CO,
d) Erwärmen des Reaktionsgemisches aus a) bis c), wobei das Olefin zu einem Aldehyd umgesetzt wird.

10. Verfahren nach Anspruch 9,
wobei die ethylenisch ungesättigte Verbindung in Verfahrensschritt a) ausgewählt ist aus: Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

11. Verfahren nach einem der Ansprüche 9 oder 10,
wobei die Substanz, welche Rh umfasst, ausgewählt ist aus: Rh(acac)(CO)₂, [(acac)Rh(COD)] (Umicore, acac = Acetylacetonat-Anion; COD = 1,5-Cyclooctadien), Rh₄CO₁₂.

12. Verfahren nach einem der Ansprüche 9 bis 11,
wobei das Zuführen von CO in Verfahrensschritt c) mit einem Druck in dem Bereich von 1 bis 6 MPa (10 bis 60 bar) erfolgt.

13. Verfahren nach einem der Ansprüche 9 bis 12,
wobei das Erwärmen des Reaktionsgemisches in Verfahrensschritt d) auf eine Temperatur in dem Bereich von 80 °C bis 160 °C erfolgt.
